# EUROPÄISCHE PATENTANMELDUNG

(11) **EP 4 617 232 A1**
(43) Veröffentlichungstag der Anmeldung: **17.09.2025**
(21) Anmeldenummer: 24163327.0
(22) Anmeldetag: 13.03.2024
(51) Int. Cl.: C01B 3/38, C10G 1/08, C10G 1/10, C10G 3/00, C10L 1/04, C07C 29/00

(54) **KATALYTISCH ERZEUGTE ALKANE**

(71) Anmelder: Fehrenbach, Sebastian, 08412 Werdau (DE); Mädel, Jürgen, 07806 Neustadt an der Orla (DE)
(72) Erfinder: Fehrenbach, Sebastian, 08412 Werdau (DE); Mädel, Jürgen, 07806 Neustadt an der Orla (DE); Stafiej, Jan, 56112 Lahnstein (DE)
(74) Vertreter: Kayser, Christoph

(57) **Zusammenfassung**

Die vorliegende Erfindung bezieht sich auf ein Verfahren zur Herstellung einer Mischung aus Alkanen; eine Mischung aus Alkanen, die gemäß dem erfindungsgemäßen Verfahren hergestellt wird; ein Verfahren zur Herstellung von Methanol; ein Verfahren zur Herstellung von Methan; ein Verfahren zur Herstellung von Wasserstoff unter Verwendung der erfindungsgemäß hergestellten Alkane; und eine Vorrichtung zur Herstellung einer Mischung aus Alkanen.

## Beschreibung

### Gegenstand der Erfindung

Die vorliegende Erfindung bezieht sich auf ein Verfahren zur Herstellung einer Mischung aus Alkanen; eine Mischung aus Alkanen, die gemäß dem erfindungsgemäßen Verfahren hergestellt wird; ein Verfahren zur Herstellung von Methanol; ein Verfahren zur Herstellung von Methan; ein Verfahren zur Herstellung von Wasserstoff unter Verwendung der erfindungsgemäß hergestellten Alkane; und eine Vorrichtung zur Herstellung einer Mischung aus Alkanen.

### Stand der Technik

Im Zuge der globalen Bestrebungen, nachhaltige Lösungen für die Energiegewinnung und das Abfallmanagement zu entwickeln, hat die Umwandlung von Biomasse, einschließlich organischer Abfälle, in biobasierte flüssige Brennstoffe, wie Öle und Diesel, sowie die gleichzeitige Rückgewinnung wertvoller Mineralien zur Düngemittelproduktion, an Bedeutung gewonnen. Diese Ansätze ermöglichen nicht nur die Verringerung der Abhängigkeit von fossilen Brennstoffen durch die Bereitstellung erneuerbarer Energiequellen, sondern fördern auch einen geschlossenen Kreislauf, indem Abfallprodukte in nützliche und speicherbare Ressourcen umgewandelt werden.

Die katalytische Reaktion von Rohstoffen und Reststoffen zu Mitteldestillat in sogenannten Reaktionsturbinen ist bekannt. Ein Nachteil der Erfindung und die gleichzeitige Ursache für die derzeitige geringe Anwendung dieser Technologie beruht in dem hohen Stromverbrauch der Reaktionsturbinen, in denen die Aufwärmung und die Vermischung der Eingangsstoffe mit dem Reaktionsöl und dem Katalysator vorgenommen wird. Durch diesen hohen Stromverbrauch wird ein Großteil des erzeugten Produkts wieder für die Herstellung von Strom benötigt. Es werden bis zu 30% des erzeugten Produkts wieder für die Herstellung für Strom benötigt. Ein weiterer Nachteil der Verwendung von Reaktionsturbinen liegt in deren Schallentwicklung und dem regelmäßigen hohen Serviceaufwand.

Die vorliegende Patentanmeldung beschreibt eine effiziente und nachhaltige Methode zur Umwandlung von Biomasse, deren Kern darauf beruht, einen integrierten Prozess zur Umwandlung von Biomasse in hochwertige Brennstoffe und Ausgangschemikalien und die Umwandlung in der Biomasse enthaltenen Mineralien zu Düngemitteln zur Verfügung zu stellen. Darüber hinaus ist das Verfahren gemäß der vorliegenden Erfindung ebenfalls energieeffizient.

Es ist daher eine Aufgabe der vorliegenden Erfindung, ein Verfahren zur Umwandlung von Biomasse in hochwertige Brennstoffe und Düngemittel zur Verfügung zu stellen, das wesentlich energieeffizienter betrieben werden kann als die im Stand der Technik bekannten Verfahren.

### Zusammenfassung

Die Erfinder der vorliegenden Erfindung haben gefunden, dass der hohe Stromverbrauch bei der Umwandlung von Biomasse zu Mischungen aus Alkanen verringert werden kann, indem das Verfahren in einem Injektionsrührbehälter (1) unter Injektion von Luft bzw. Sauerstoff durchgeführt wird. Der verringerte Energieverbrauch beruht darauf, dass die Erwärmung auf die Verfahrenstemperatur sowie die Erwärmung der Ausgangsstoffe auf die Verfahrenstemperatur durch partielle Oxidation in der Flüssigphase stattfindet, so dass der Stromverbrauch des Verfahrens um mindestens 95% gesenkt wird. Die benötigte Energie zur Erwärmung der Ausgangsstoffe von 20°C auf die Verfahrenstemperatur von 300°C wird nicht mehr durch die Reibung in Reaktionsturbinen erzeugt, sondern fast ausschließlich durch die partielle Oxidation mit einem Gas, das Sauerstoff enthält, wie beispielsweise Luft oder reiner Sauerstoff.

Daher bezieht sich die vorliegende Erfindung in einem Aspekt auf ein Verfahren zur Herstellung einer Mischung aus Alkanen, das wenigstens die folgenden Schritte umfasst:
(A) Umsetzen von Biomasse mit wenigstens einem Gas, das Sauerstoff enthält, in Anwesenheit wenigstens eines Katalysators in der Flüssigphase bei einer Temperatur im Bereich von ≥ 270 bis ≤ 360 °C, bevorzugt bei 300 °C, um eine Mischung aus Reaktionsgas, Mischung aus Alkanen und Wasserdampf zu erhalten, und
(B) Abtrennen der Mischung aus Alkanen von Reaktionsgas und Wasserdampf durch Kondensation in einem Produktdampfseparator (2), um die Mischung aus Alkanen zu erhalten,
dadurch gekennzeichnet, dass Schritt (A) in einem Injektionsrührbehälter (1) erfolgt.

Das erfindungsgemäße Verfahren zeichnet sich dadurch aus, dass der Katalysator die Biomasse, d.h. die Roh- und Reststoffe, aktiviert. Roh- und Reststoffe können organische Abfälle sein, aber auch Kunststoffe wie Polyester, wie PET, und Polyalkylene, wie beispielsweise Polyethylen und Polypropylen. Durch die Oxidation der Biomasse wird CO₂ gebildet, das ebenfalls die Reaktionsgeschwindigkeit erhöht. Vorzugsweise wird die Biomasse in dem Injektionsrührbehälter (1) innerhalb weniger Minuten, bevorzugt innerhalb von ≥ 2 bis ≤ 10 Minuten umgesetzt. Der Injektionsrührbehälter ist damit nicht nur der Reaktor für die Umsetzung der Biomasse, sondern gewährleistet auch die Bereitstellung der Energie für den gesamten Prozess, d.h. die Folge aus Oxidation, selektiver Kondensation der Alkane, Destillation der Alkane und Abscheiden der Reaktionsgase und Kondensation des Wasserdampfs.

Der Injektionsrührbehälter (1) umfasst einen Tank (1d), wenigstens einen Rührer (1a) und wenigstens zwei Einlässe (1b, 1c) für Gase, wobei der wenigstens eine Rührer (1a) im unteren Bereich des Tanks (1d) angeordnet ist.

Der Injektionsrührbehälter (1) kann ein Prallmischer oder eine Pralllmischturbine sein.

Die Umsetzung von Biomasse zu einer Mischung aus Alkanen erfolgt in einem Reaktions- bzw. Rührbehälter, der im Folgenden als Injektionsrührbehälter (1) bezeichnet wird. Der Injektionsrührbehälter (1) ist zum Teil mit einer Flüssigphase gefüllt, die aus der Biomasse in flüssiger Form und dem Katalysator besteht.

Der Kern des Injektionsrührbehälters (1) besteht aus einem Tank (1d), der aus Materialien wie Edelstahl, Glasfaser oder speziellen Kunststoffen gefertigt ist, um chemische Beständigkeit, Reinheit und Langlebigkeit zu gewährleisten. Innerhalb des Tanks (1d) befindet sich wenigstens ein Rührer (1a), der in der Regel aus einem oder mehreren Rührern (1a) besteht, wie beispielsweise Propeller-, Turbinen-, Paddel- oder Ankerrührern, die für eine homogene Mischung der Inhaltsstoffe, bevorzugt der Flüssigphase, sorgen. Ein Injektionsrührbehälter (1) kann in verschiedenen Formen und Größen konstruiert sein, um den spezifischen Anforderungen des Verfahrens zu genügen. Die Wahl der Form und Größe des Injektionsrührbehälters (1) hängt von vielen Faktoren ab, darunter das Volumen der zu verarbeitenden Materialien, die Art der chemischen Reaktion oder Mischung, die physikalischen Eigenschaften der Biomasse und des Katalysators und die erforderliche Verarbeitungszeit.

Der Injektionsrührbehälter (1) liegt vorzugsweise in zylindrischer Form, kugelförmiger Form oder konischer Form vor. Bevorzugt liegt der Injektionsrührbehälter (1) in einer zylindrischen Form vor, die einen runden Querschnitt aufweist. Diese Form bietet eine gute Balance zwischen Volumeneffizienz und Oberfläche für den Wärmeaustausch und erleichtert die Reinigung und Wartung.

Der Rührer (1a) ist bevorzugt ein Rührer vom Typ Propellerrührer, Turbinenrührer, Paddelrührer oder Ankerrührer. Der Rührer (1a) gewährleistet die optimale Mischung der Flüssigphase mit dem Gas. Propellerrührer bestehen aus zwei oder mehr Blättern, die an einer zentralen Achse befestigt sind. Sie drehen sich schnell und erzeugen einen axialen Fluss, der Flüssigkeiten entlang der Achse des Tanks (1d) bewegt. Propellerrührer werden bevorzugt, um das Mischen von niedrigviskosen Flüssigkeiten und das Dispergieren von Gasen in Flüssigkeiten zu gewährleisten. Weiterhin bevorzugt sind Turbinenrührer. Turbinenrührer bestehen aus mehreren flachen oder gekrümmten Blättern, die radial um eine zentrale Achse angeordnet sind. Sie erzeugen einen radialen Fluss, der Flüssigkeiten quer durch den Tank (1d) treibt und für intensive Mischwirkung sorgt. Weitere bevorzugte Rührer sind vom Typ Paddelrührer oder Ankerrührer. Paddelrührer haben eine einfache Konstruktion mit einem oder mehreren flachen Blättern, die senkrecht zur Rührachse angebracht sind. Sie bewegen sich in der Regel langsam und erzeugen einen sanften Mischvorgang mit geringer Scherkraft. Ankerrührer weisen große, flache Blätter auf, die der Innenkontur des Tanks (1d) folgen. Sie drehen sich langsam und sorgen für ein gründliches Mischen durch Schaben der Tankwände, was die Bildung von Totzonen verhindert.

Weiterhin umfasst der Injektionsrührbehälter wenigstens zwei Einlässe (1b, 1c). Einlässe für Gase in einen Tank (1d) sind speziell konstruierte Öffnungen oder Vorrichtungen, die es ermöglichen, Gase oder gasförmige Substanzen kontrolliert in den Innenraum eines Tanks (1d) einzuleiten. Die Einlässe können in Form von Spargern, Gasinjektionsdüsen und Belüftungsventilen vorliegen. Ein Sparger ist eine Vorrichtung, die am Boden oder an der Seite eines Tanks (1d) angebracht wird, um Gas direkt in die Flüssigkeit einzuleiten. Ein Sparger verteilt das Gas in feine Blasen, wodurch die Kontaktfläche zwischen Gas und Flüssigkeit maximiert und die Effizienz des Gasaustausches erhöht wird. Gasinjektionsdüsen sind konstruierte Öffnungen, die das Gas in bestimmten Mustern oder unter spezifischen Druckbedingungen in den Tank (1d) einleiten. Sie können so gestaltet sein, dass sie das Gas in Form eines gezielten Strahls oder einem diffusen Muster für eine breite Verteilung einspeisen. Belüftungsventile ermöglichen das konstruierte Ein- und Auslassen von Gasen, um den Druck innerhalb des Tanks (1d) zu regulieren oder eine bestimmte Atmosphäre zu schaffen. Diese Ventile können manuell oder automatisch gesteuert werden, um auf Veränderungen im Prozess zu reagieren.

In einer bevorzugten Ausführungsform ist einer der wenigstens zwei Einlässe (1b) oberhalb des Rührers (1a) angeordnet und einer der wenigstens zwei Einlässe (1c) ist unterhalb des Rührers (1a) angeordnet. Bevorzugt wird das wenigstens eine Gas, das Sauerstoff enthält, über den wenigstens einen ersten Einlass (1b) angesaugt, wobei das Gas vorgewärmt ist, und das wenigstens eine Gas, das Sauerstoff enthält, wird bevorzugt unter Druck über den wenigstens einen zweiten Einlass (1c) zugeführt. Dabei erfolgt die Luftvorwärmung im oberen Teil drucklos und ist verbunden mit der Ansaugung der heißen Luft oberhalb des Rührers (1a) im Injektionsrührbehälter (1). Im unteren Teil wird das Gas mit Druck aus dem Verdichter, der die Luft unterhalb des Rührers (1a) über einen Blasenring eindüst, zugeführt.

In einer bevorzugten Ausführungsform weist der Injektionsrührbehälter (1) einen Doppelmantel auf, der ein Thermoöl beinhaltet. Der Doppelmantel umschließt den Injektionsrührbehälter und ist für die Zirkulation von Thermoöl konzipiert. Der Doppelmantel ist so konstruiert, dass er eine optimale geometrische Form bietet, welche die Effizienz der Wärmeübertragung maximiert. Die geometrische Konfiguration des Doppelmantels ist präzise auf die Dimensionen des Tanks (1d) abgestimmt, um eine gleichmäßige Verteilung des Thermoöls rund um den Innenraum des Tanks (1d) zu gewährleisten. Das in den Doppelmantel eingefüllte Thermoöl wurde speziell ausgewählt, um eine hohe thermische Leitfähigkeit und eine stabile thermische Performance über einen breiten Temperaturbereich hinweg zu bieten. Diese Eigenschaften des Thermoöls ermöglichen eine schnelle und gleichmäßige Wärmeübertragung. Die Auswahl des Thermoöls basiert auf dessen Fähigkeit, Wärme effizient zu absorbieren und abzugeben.

In einer bevorzugten Ausführungsform ist der wenigstens eine Katalysator ein Kation-Aluminium-Silikat. Kation-Aluminium-Silikate sind Zeolithe. Zeolithe sind mikroporöse, kristalline Alumosilikate, die aus einem dreidimensionalen Gerüst von SiO₄- und AlO₄-Tetraedern bestehen, die durch Sauerstoffatome miteinander verbunden sind. Die Substitution von Si(IV) durch Al(III) im Kristallgitter führt zu einer negativen Ladung, die durch die Aufnahme von Kationen wie Na⁺, K⁺, Ca²⁺ ausgeglichen wird. Diese Kationen sind austauschbar, was Zeolithen die Fähigkeit verleiht, selektiv Moleküle auf Basis ihrer Größe und elektrischen Ladung zu adsorbieren oder zu desorbieren. Die Zeolithe, das heißt das Kation-Aluminium-Silikat, gewährleisten die spezifische Umwandlung der Biomasse zu Mischungen aus Alkanen.

In einer bevorzugten Ausführungsform wird ein kleiner Teil der Flüssigphase kontinuierlich im Verfahren in einem Inertgasvakuumverdampfer (7) recycliert, wobei ein fester Rückstand abgetrennt wird. Der Inertgasvakuumverdampfer (7) wird mit Thermoöl über den Injektionsrührbehälter (1) geheizt und steht unter Vakuum, wodurch die entsprechende Menge der Flüssigphase für die Rezyklisierung über ein Ventil angesaugt wird. Über ein Inertgas und die Einstellung des Vakuums wird der Partialdruck der Flüssigphase abgesenkt. Das führt dazu, dass die Flüssigphase auch bei 300° C nahezu vollständig verdampft und am Boden die anorganischen Bestandteile zurückbleiben. Diese anorganischen Bestandteile werden gemäß der vorliegenden Erfindung in Wasser suspendiert. Die unlösliche Katalysatorsubstanz Calcium-Aluminium-Silikat wird im Wasserkreislauf ausgeschieden und dem Eintragssystem der Anlage zugeführt. Die Sole wird nach ihrer Reinigung in der selektiven Elektrolyse eingedampft und kann als Dünger verwendet werden. Die selektive Elektrolyse nutzt niedrige Voltzahlen, um an Elektroden metallische Verunreinigungen, beispielsweise aus Medikamenten, abzuscheiden und als Produkt zu nutzen.

In einer bevorzugten Ausführungsform umfasst der Injektionsrührbehälter (1) wenigstens einen Auslass (1e). Die Mischung aus Reaktionsgas, Mischung aus Alkanen und Wasserdampf wird bevorzugt über wenigstens einen Auslass (1e) und einen 180° Bogen (2a) und wenigstens einen Wärmeaustauscher (2b), bevorzugt zwei Wärmeaustauscher (2b), in die Einspritzzone des Produktdampfseparators (2) eingeführt, um die Temperatur für die nachfolgende selektive Kondensation auf 100° C abzusenken. Die Kondensation erfolgt bevorzugt in dem Produktdampfseparator (2) bei einer Temperatur im Bereich von ≥ 90 bis ≤ 110 °C, bevorzugt bei 100 °C. Das katalytisch erzeugte Gemisch aus Reaktionsgas, Mischung aus Alkanen und Wasserdampf gelangt in einem fallenden Rohr in die Einspritzzone des Produktdampfseparators (2), der die Mischung aus Alkanen kondensiert, den Wasserdampf und andere Reaktionsgase jedoch passieren lässt. Dazu wird das Produkt aus dem unteren Verbindungsrohr zwischen dieser partiellen Kondensation und der Destillation eingespritzt, um die Temperatur auf 100°C abzusenken. Bei Bedarf erfolgt eine zusätzliche Einspritzung von kondensiertem Wasser. Damit wird sichergestellt, dass nur das Produkt (das heißt, die Mischung aus Alkanen), in dem Produktdampfseparator (2) verbleibt und die Wasserdampfanteile und andere Reaktionsgase den Behälter verlassen. Damit bildet sich in dem Produktdampfseparator (2) eine ansteigende Menge von einer Mischung aus Alkanen. Der Produktdampfseparator (2) ist im unteren Bereich mit dem Produktdestillationsbehälter (3) verbunden, der im oberen Teil mit Thermoöl geheizt wird. Wird diese obere geheizte Zone mit dem Füllstand erreicht, verdampft die saubere Mischung aus Alkanen. Die Mischung aus Alkanen hat durch ihren chemischen Aufbau mit den Seitenketten auch eine niedrigere Verdampfungstemperatur von ca. 200°C. Dadurch reicht die Thermoölheizung des Mantels an dem Produktdestillationsbehälter (3) völlig aus, um die Mischung aus Alkanen zu verdampfen. Zusätzlich ist an der Destillationsanlage nach der Kondensation in der Kolonne und dem Kondensator eine Vakuumanlage an dem Produkttank angeschlossen, die die Verdampfung der Mischung aus Alkanen unterstützt und eine niedrigere Verdampfungstemperatur ermöglicht. Beheizt wird der Produktdestillationsbehälter (3) über den Injektionsrührbehälter (1) und eine entsprechende Thermoöl-Mantelvorrichtung für einen Thermoölkreislauf zwischen dem Mantel des Injektionsrührbehälters (1) zu dem oberen Teil des Produktdestillationsbehälters (3) und dem ersten Teil der Kolonne der Destillationsanlage. Mit der Temperatur des Thermoöls, welches sich im Injektionsrührbehälter (1) mit fast 300 °C einstellt, ist die kontinuierliche Destillation der Mischung aus Alkanen gesichert.

Der Injektionsrührbehälter (1) weist ebenfalls eine Eintragsschnecke auf, die die Biomasse von oben über einen Zufuhrschacht bis unter den Flüssigkeitsspiegel der Flüssigphase im Tank (1d) einträgt. Der Rührer (1a) erfasst die Biomasse und vermischt diese in der Flüssigphase. Die Verweilzeit der Biomasse im Injektionsrührbehälter (1) von deren Eintrag bis zur chemischen Zersetzung und Umwandlung in Alkane, Wasserdampf und Reaktionsgas beträgt je nach Menge wenige Minuten. Sofern die Biomasse Säurebildner enthält, werden diese durch die zusätzliche Zugabe von ungefähr 1 % Kalkstein (CaCOs) neutralisiert.

Die aus dem Produktdampfseparator (2) abgezogenen Wasserdämpfe und weitere Reaktionsgase aus der partiellen Oxidation gelangen in einen Wasserkondensationsbehälter (5) und werden dort auch durch luftgekühltes Kreislaufwasser und Einspritzen dieses Wassers zur Kondensation in flüssiges Wasser und Inertgas separiert. Das Inertgas wird zu einem kleinen Teil von dem Inertgasvakuumverdampfer (7) angesaugt und zum größten Teil über einen Keramikfilter (6) gereinigt nach außen abgegeben.

Das kondensierte Wasser aus dem Wasserkondensationsbehälter (5) gelangt in einen Produktwassertank (4), der über den Keramikfilter (6) einen leichten Unterdruck durch eine Vakuumpumpe aufweist. In diesem Produktwassertank (4) bildet sich eine schwimmende Schicht aus einer Mischung aus Alkanen aus, die bei unter 100°C kondensiert. Durch Schwerkraftabscheidung wird dieser Anteil separiert und über eine Flüssigpumpe dem Eintragssystem oder der Destillationsanlage im unteren Verbindungsrohr zugeführt.

Das erfindungsgemäße Verfahren ermöglicht die partielle Oxidation von Biomasse, wie organischen Abfällen und Kunststoffen, wie Polyestern einschließlich PET und Polyalkylenen einschließlich Polyethylen und Polypropylen, zu einer Mischung aus Alkanen.

In einem weiteren Aspekt bezieht sich die vorliegende Erfindung daher auf eine Mischung aus Alkanen mit der Formel CₙH₂ₙ₊₂, wobei n = 14, 15, 16, 17, 18, 19, 20, 21, 22 oder 23 ist. Bevorzugt ist n im Mittel 17. Die Moleküllänge der Alkane, also die Zahl n, variiert mit der Prozesstemperatur.

Die Mischung aus Alkanen, die gemäß dem erfindungsgemäßen Verfahren erhalten wurde, kann weiter zu Methanol, Methan oder Wasserstoff umgesetzt werden. Die Umsetzung erfolgt in einem nachfolgenden Röhrenbündeldruckreaktor (8), der mit dem Thermoöl über den Injektionsrührbehälter (1) geheizt wird. In dem Röhrenbündeldruckreaktor (8) befindet sich eine Rohrschlange von oben nach unten und nach einer Trennwand mit Durchlass von unten nach oben. Im Gegenstrom wird das Thermoöl von etwa 300 °C um die Rohrschlangen geleitet und erwärmt diese entsprechend. In den Rohrschlangen befindet sich ein Gestrick von Aluminium, das mit dem Katalysator CuZnOAl₂O₃ beschichtet ist, und durch Tränken und anschließender Kalzinierung aufgebracht wurde. Die Mischung aus Alkanen wird nun mit Wasser und suspendiertem Aluminium-Silikat, bevorzugt Kation-Aluminium-Silikat, zur Aktivierung des Kohlenstoffs gemischt, unter einem Druck von 200 bar in das Rohrbündel eingepumpt und am Ende nach Abkühlung im folgenden Wärmetauscher und Kühler entspannt.

Entsprechend bezieht sich die vorliegende Erfindung auch auf ein Verfahren zur Herstellung von Methanol, wobei die erfindungsgemäße Mischung aus Alkanen mit Wasser gemäß der Formel CₙH₂ₙ₊₂ + n × H₂O = n × CH₃OH umgesetzt wird, wobei n = 14, 15, 16, 17, 18, 19, 20, 21, 22 oder 23 ist.

Ebenfalls bezieht sich die vorliegende Erfindung auf ein Verfahren zur Herstellung von Methan, wobei eine Mischung aus erfindungsgemäß erhaltenen Alkanen mit Wasser gemäß der Formel 2 CₙH₂ₙ₊₂ + 2 × 1,5 n × H₂O = 2n × CH₄ + n CO₂ umgesetzt wird, wobei n die vorstehend genannte Bedeutung hat.

Ebenfalls bezieht sich die vorliegende Erfindung auf ein Verfahren zur Herstellung von Wasserstoff, wobei die erfindungsgemäß erhaltene Mischung aus Alkanen gemäß der Formel CₙH₂ₙ₊₂ + 2n × H₂O = 3n × H₂ + n CO₂ umgesetzt wird, wobei n die vorstehend genannte Bedeutung hat.

Das gewünschte Produkt kann somit durch Einstellung des Mischungsverhältnisses von Alkanen zu Wasser bestimmt werden. Die Länge des Reaktors und das Mischungsverhältnis mit dem Katalysator steigen von der Erzeugung von Methanol über die Erzeugung von Methan zu Wasserstoff an, um die vollständige Umsetzung der Mischung aus Alkanen zu gewährleisten.

In einem weiteren Aspekt bezieht sich die vorliegende Erfindung auf eine Mischung aus Alkanen mit der Formel CₙH₂ₙ₊₂, wobei n = 14, 15, 16, 17, 18, 19, 20, 21, 22 oder 23 ist, die eine Dichte bei 15 °C im Bereich von ≥ 740 bis ≤ 850 kg/m³, bestimmt nach DIN EN ISO 12185:1997, und
einen Flammpunkt im Bereich von ≥ 30 bis ≤ 65 °C, bestimmt nach DIN EN ISO 2719:2021, und
einen Schwefelgehalt im Bereich von ≥ 5 bis ≤ 500 mg/kg, bestimmt nach DIN EN ISO 20884:2022, und
eine Säurezahl im Bereich von ≥ 100 bis ≤ 110 mg KOH/g, bestimmt nach DIN EN ISO 14104:2003, aufweist.

Weiterhin weist die Mischung aus Alkanen bevorzugt eine kinetische Viskosität im Bereich von ≥ 1,8²/s bis ≤ 8,8 mm²/s, bestimmt nach DIN EN ISO 3104:2021 auf. Weiterhin weist die Mischung aus Alkanen bevorzugt einen Cold Filter Plugging Point (CFPP) im Bereich von ≥ -30 °C bis ≤ -10 °C auf, bestimmt nach DIN EN 116:2018. Weiterhin weist die erfindungsgemäße Mischung aus Alkanen bevorzugt eine Cetanzahl (ICN) im Bereich von ≥ 45 bis ≤ 65 auf, bestimmt nach DIN EN 17155:2018. Ebenfalls bevorzugt weist die erfindungsgemäße Mischung aus Alkanen bevorzugt einen Gehalt von Sulfatasche (775 °C) von <0,005% (m/m) auf, bestimmt nach ISO 3987:2010. Weiterhin weist die erfindungsgemäße Mischung aus Alkanen bevorzugt einen Wassergehalt im Bereich von ≥ ≤ 3600 mg/kg, vorzugsweise sogar ≤ 200mg/kg auf, bestimmt nach DIN 51777 Verf. A:2020. Weiterhin weist die erfindungsgemäße Mischung aus Alkanen bevorzugt einen Alkaligehalt (Na+K) von <1,0 mg/kg auf, bestimmt nach DIN EN 14538:2006 und einen Metallgehalt II (Ca+Mg) von <1,0 mg/kg auf, bestimmt nach DIN EN 14538:2006. Weiterhin bevorzugt weist die erfindungsgemäße Mischung aus Alkanen einen Cloud Point im Bereich von ≥ -15 °C bis ≤ -5 °C auf, bestimmt nach DIN EN 23015:1994.

In einem weiteren Aspekt bezieht sich die vorliegende Erfindung ebenfalls auf eine Vorrichtung zur Herstellung einer Mischung aus Alkanen umfassend
wenigstens einen Injektionsrührbehälter (1), der einen Tank (1d), wenigstens einen Rührer (1a), wenigstens einen Auslass (1e) und wenigstens zwei Einlässe (1b, 1c) für Gase umfasst, wobei der wenigstens eine Rührer (1a) im unteren Bereich des Tanks (1d) angeordnet ist,
wenigstens einer der zwei Einlässe (1b) oberhalb des Rührers (1a) angeordnet ist, und einer der wenigstens zwei Einlässe (1c) unterhalb des Rührers (1a) angeordnet ist, und
einen Produktdampfseparator (2), wobei die Wärmeaustauscher (2b) zwischen dem wenigstens einen Auslass (1e) und dem Produktdampfseparator (2) angeordnet sind.

In einer bevorzugten Ausführungsform der Vorrichtung wird das wenigstens eine Gas, das Sauerstoff enthält, über den wenigstens einen ersten Einlass (1b) angesaugt, wobei das Gas durch Wärmeaustauscher (2b) vorgewärmt ist, und das wenigstens eine Gas, das Sauerstoff enthält, mit Druck über den wenigstens einen zweiten Einlass (1c) zugeführt wird, wobei der zweite Einlass (1c) mit einem Wärmeaustauscher (2c) und einem Luftverdichter (1f) verbunden ist.

Die vorstehend und nachstehend beschriebenen bevorzugten Ausführungsformen des Verfahrens beziehen sich ebenfalls auf die Vorrichtung.

### Detaillierte Beschreibung der Ausführungsformen

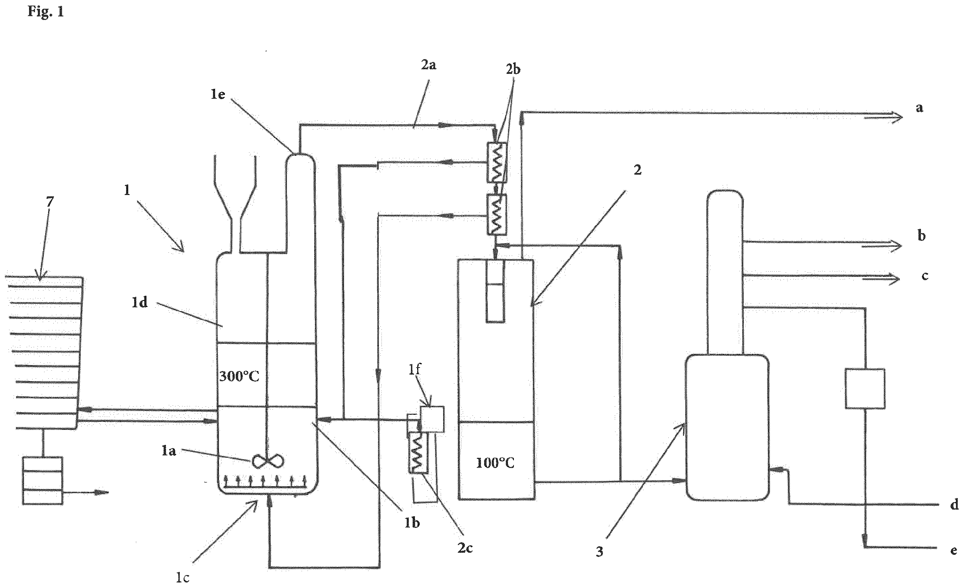

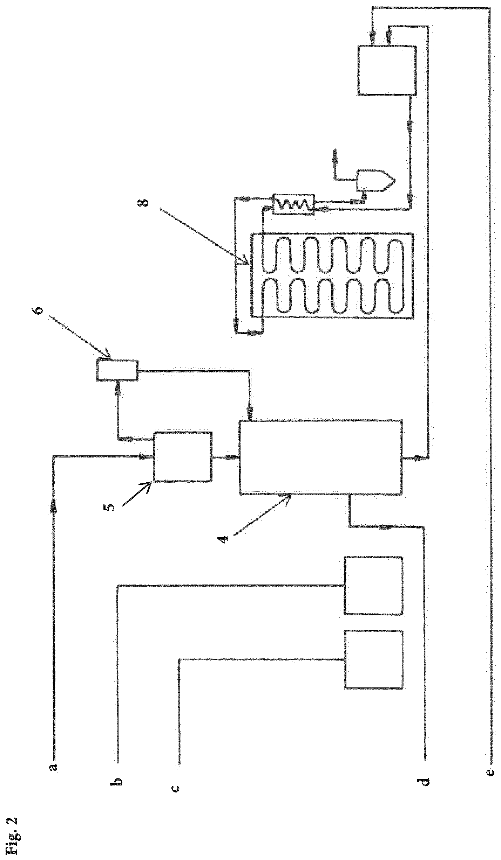

Die folgenden Bezugszeichen werden in Fig. 1 und Fig. 2 verwendet. Fig. 1 zeigt einen erfindungswesentlichen linken Teil der erfindungsgemäßen Anlage und Fig. 2 zeigt einen erfindungswesentlichen rechten Teil der erfindungsgemäßen Anlage, wobei die jeweilige Anknüpfung durch die Buchstaben a, b, c, d und e gekennzeichnet ist.

### Bezugszeichenliste:

- 1: Injektionsrührbehälter
- 1a: Rührer
- 1b: Einlass
- 1c: Einlass
- 1d: Tank
- 1e: Auslass
- 1f: Luftverdichter
- 2: Produktdampfseparator
- 2a: Bogen
- 2b: Wärmetauscher
- 2c: Wärmetauscher
- 3: Produktdestillationsbehälter
- 4: Produktwassertank
- 5: Wasserkondensationsbehälter
- 6: Keramikfilter
- 7: Inertgasvakuumverdampfer
- 8: Röhrenbündeldruckreaktor

Der Injektionsrührbehälter (1) besitzt einen Heißlufteintrag durch den Rührer von dem oberen Teil des Wärmetauschers oberhalb der selektiven Kondensation des Produktdampfseparators (2), sowie über einen Verdichter, der mit dem unteren Teil des Wärmetauschers oberhalb des Produktdampfseparators (2) verbunden ist und die Luft unterhalb des Rührers einbläst.

Dieser Behälter, der Produktdampfseparator (2) der selektiven Kondensation mit dem Einspritzsystem des Produktes und bei Überhitzung von zusätzlichem Produktwasser beinhaltet einen Demister, der dafür sorgt, dass die Mischung aus Alkanen in flüssiger Form gewonnen wird und sich am Boden dieses Behälters ansammelt. Dieser Boden des Produktdampfseparators (2) der selektiven Kondensation ist mit einem Verbindungsrohr mit dem Produktdestillationsbehälter (3) (partielle Rektifikation) verbunden, aus dem auch die Produkteinspritzung für den Produktdampfseparator (2) über eine Pumpe erfolgt.

Das Niveau im Produktdampfseparator (2) erhöht sich durch die Produktion und lässt somit auch das Niveau im Produktdestillationsbehälter (3) ansteigen, wodurch bei entsprechender Füllmenge eine Verdampfung erfolgt.

Zwischen dem Produktdestillationsbehälter (3) und dem Produktdampfseparator (2) der selektiven Kondensation ist der Verdichter für die Einblasluft angeordnet. Dieser besitzt für den Fall einer Sauerstoffanreicherung zwei Molekularsiebe für eine Druckwechseladsorption.

Über dem Produktwassertank (4) wird über eine Einspritzung von gekühltem Wasser im Wasserkondensationsbehälter (5) der Wasseranteil von dem Inertgas aus dem Produktdampfseparator (2) getrennt.

Im Produktwassertank (4) gibt es eine Schwerkraftabscheidung für eine Produktseparation von restlichen Mengen der Mischung aus Alkanen, die separiert und dem Eintragssystem des Injektionsrührbehälters (1) oder dem Produktdestillationsbehälter (3) zugeführt werden. Das von dem Wasser abgetrennte Inertgas gelangt über einen Keramikfilter (6) zu einem kleinen Teil in den Inertgasvakuumverdampfer (7) und zum großen Teil wird es an die Luft abgegeben.

Der Anteil des Inertgases, der von dem Inertgasvakuumverdampfer (7) angesaugt wird, erfährt eine Aufheizung durch die Teilabkühlung des oben abgezogenen Produktes aus Öldampf und Inertgas aus dem Inertgasvakuumverdampfer (7) in einem nachfolgenden Wärmetauscher. Diesem Wärmetauscher ist ein Kühler nachgeschaltet.

Der Inertgasvakuumverdampfer (7) separiert somit den Ölanteil, welcher zurück in die Anlage geführt wird, vom am unteren Ende anfallenden anorganischen Granulat. Dieses gelangt durch die Schneckenschleuse am unteren Ende in den darunter angeordneten Behälter zum Separieren der Düngemittel/Mineralsalze von dem Katalysator. Das gelingt mit dem zugeführten Wasser aus dem Produktwassertank (4), der den unlöslichen Teil, den Katalysator für das Recycling von der Sole trennt. Aus der Sole werden nach einer selektiven Elektrolyse und Eindampfen dieser die Düngemittel/Mineralsalze gewonnen.

Bei der Verarbeitung von Abfallbiomasse, der am häufigsten verfügbaren Menge an Eingangsstoff (Roh- und Reststoff), sind in dem erzeugten Düngemittel alle Mineralien vorhanden, die die Pflanze dem Boden entzogen hat. Damit kann durch die vorliegende Erfindung ebenfalls die Düngerthematik optimiert werden. Durch Sonnenenergie erzeugte Biomasse (Bildung Zellulose über Photosynthese), wird in Form einer Mischung aus Alkanen gespeichert und die dem Boden entzogenen Mineralien können dem Ackerboden oder Wald wieder zugeführt werden.

### Beispiele

Im Folgenden wird die vorliegende Erfindung anhand von Beispielen erläutert. Die Beispiele dienen lediglich der Erläuterung der Erfindung und sind in keiner Weise dazu geeignet den Gegenstand der Erfindung einzuschränken. 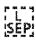 Ein Generator mit 500 kW erhitzt das Thermoöl des Heizmantels eines Injektionsrührbehälters (1) und das darin enthaltene Reaktionsöl durch Wärmetausch und elektrische Erwärmung auf 300°C. Diese Erwärmung dauert für einen Injektionsrührbehälter (1) 30 - 40 Minuten. Anschließend wird die Erwärmung über den Generator gestoppt und die Luftansaugung sowie Lufteinblasung in den Injektionsrührbehälter (1) übernimmt durch die partielle Oxidation in die Flüssigphase die weitere Erwärmung und Aufheizung der über ein Eintragssystem eingebrachten Roh- und Reststoffe wie Holz, Ernterückstände und Kunststoffe. Die Produktionsmenge von einem Injektionsrührbehälter (1) ist auf 500 Liter/h Mischung aus Alkanen ausgelegt und erfolgt durch die Ansaugung von 250 Kubikmeter Luft pro Stunde durch den Rührer und 250 Kubikmeter Luft pro Stunde über den Luftverdichter.

Nach Erhöhung der Temperatur auf 330 °C kann ein zweiter Injektionsrührbehälter (1) auf Betriebstemperatur aufgewärmt werden, indem über eine Umlaufpumpe Thermoöl von dem Mantel des ersten Behälters den zweiten Injektionsrührbehälter (1) aufwärmt. Die Leistung einer Anlage kann somit von einem Injektionsrührbehälter (1) mit 500 Liter pro Stunde auf 5000 Liter pro Stunde Mischung aus Alkanen mit 10 Injektionsrührbehältern (1) erhöht werden. Dazu werden jeweils 3-5 Injektionsrührbehälter (1) auf einen Produktdampfseparator (2) (selektive Kondensation) und einen anschließenden Produktdestillationsbehälter (3) (partielle Rektifikation) geschaltet. Die elektrische Leistung von dem Rührer eines Injektionsrührbehälters (1) beträgt 40 kW, wodurch der Generator beim Betrieb von 10 Injektionsrührbehältern (1) insgesamt 400 kW für die Rührer liefert. Die restlichen 100 kW werden von den Pumpen und der Elektronik verbraucht.

Damit liefern die 3 Produktdestillationsbehälter (bei 10 Injektionsrührbehältern) je 1 500 bis ca. 1 700 Liter pro Stunde Mischung aus Alkanen. Jeweils ein Injektionsrührbehälter (1) befindet sich in der Reinigungsstufe mit dem Inertgasvakuumverdampfer (7). Je nach Eingangsstoff entstehen zusätzlich 2 000 Liter pro Stunde Kondensationswasser.

Bei der anschließenden Umsetzung des Produktes in die weiteren Produkte Methanol, Methan und Wasserstoff wird ein Teil der erzeugten Mischung aus Alkanen mit dem jeweiligen Anteil Wasser, also mit der gleichen Menge Wasser (1:1) für Methanol, der 1,5-fachen Menge an Wasser für Methan und der doppelten Menge an Wasser für Wasserstoff mit dem Katalysator Kation-Aluminium-Silikat homogen gemischt und über eine Druckpumpe in das Rohrbündel im Röhrenbündeldruckreaktor (8) eingepresst. Das Kreislauf-Thermoöl übernimmt die Aufheizung für die endotherme Reaktion und nach dem Röhrenbündeldruckreaktor (8) ist ein Wärmetauscher für die Aufheizung des eingepressten Flüssigkeitsgemisches und ein Kühler angeordnet. Nach dem Kühler setzt sich der Katalysator ab, der recycelt wird.

Ein Ausführungsbeispiel für die Vorrichtung soll die Erfindung näher beschreiben. Der Injektionsrührbehälter (1) hat einen Durchmesser von 1 200 mm und einen Rührer mit dem Durchmesser von 400 mm. Um den Rührer ist ein Strömungsring von 600 mm Durchmesser angebracht. Der Rührer ist mit einem elektrischen Motor von 40 kW verbunden. Am oberen Ende des Injektionsrührbehälters (1) ist ein Eintragsrohr mit einem Durchmesser von 150 mm angebracht, welches auf der Eintragsseite mit einem Schneckensystem mit Trichter und auf der Behälterseite mit einem Rohr von 200 mm verbunden ist, welches in dem 2 500 mm hohen Behälter mit einer Länge von 1 500 mm hineinragt. Auf der Ausgangsseite des Injektionsrührbehälters (1) ist ein Rohr nach oben mit 300 mm Durchmesser angebracht, welches 1 500 mm nach oben führt und dann einen 180° Bogen nach unten besitzt. Daran angeschlossen ist der drucklose Luftvorwärmer mit Rippenrohren, wobei die Rippen auf der Luftseite sind. Der danach angeschlossene Wärmeaustauscher ist druckfest auf 1 bar Überdruck und besitzt ebenfalls Rippenrohre mit Rippen auf der Luftseite.

Der Produktdampfseparator (2) besitzt in der Mitte den Demister, der an das Rohr mit D= 300 mit 500 mm Durchmesser angebracht ist. Der Produktdampfseparator (2) hat einen Durchmesser von 1 800 mm und eine Höhe von 3 000 mm. Am unteren Ende ist das Verbindungsrohr mit D= 150 mm angebracht. Die Einspritzdüsen (zum Kühlen) von in diesem Behälter erzeugten Produkt sind für 1 000 Liter pro Stunde Produkt und für 200 Liter pro Stunde Wasser ausgelegt. Der Produktdestillationsbehälter (3) hat einen Behälterdurchmesser von 1 000 mm und darüber hinaus einen Kolonnendurchmesser von 300 mm. Die Kolonne ist gefüllt mit Schütt-Füllkörpern. Die Höhe der Destillationsanlage auf dem Produktdestillationsbehälter (3) beträgt 2 500 mm.

Der Wasserkondensationsbehälter (5) ist für 2 000 Liter pro Stunde Wasser ausgelegt und hat einen Rückkühler von 2 000 kW. Der anschließende Produktwassertank (4) hat einen Durchmesser von 1 200 mm. Dieser besitzt am unteren Ende ein Schwerkraftventil, welches sich öffnet bei einer Dichte der Flüssigkeit von unter 0,95 kg/Liter und dann schließt (Rückführung Produkt in die Destillation). Nach dem Injektionsrührbehälter (1) ist der Inertgasvakuumverdampfer (7) angeordnet. Er hat einen Durchmesser von 800 mm und eine Höhe von 2 000 mm. Er ist druckdicht und am oberen Ende ist ein Getriebemotor mit 40 kW Antriebsleistung für den Rührer angebracht. Am unteren Ende ist eine druckdichte Schneckenschleuse angebracht, die auf einem Wasserbehälter von 300 Liter Inhalt sitzt. Dieser Wasserbehälter ist verbunden mit einer handelsüblichen Siebkreislaufmaschine, die auf dem Sieb den unlöslichen Katalysator in einen Zwischenbehälter ableitet, der eine Förderpumpe zu der Eintragsschnecke am Injektionsrührbehälter (1) hat. Das Kreislaufwasser, das mit dem Granulat eine Sole bildet, wird in einen Verdampfer geleitet, der mit dem Auspuffgas des Stromgenerators verbunden ist und geheizt wird. Dort wird das Düngemittelgranulat erzeugt. Vorher kann die Sole durch die selektive Elektrolyse geleitet werden.

Die Vorrichtung besteht somit aus 5 Hauptelementen 1-4 und 7 und den dazu notwendigen Hilfselementen. Für die Erzeugung von 5 000 Liter/h Mischung aus Alkanen sind das dann 10 Injektionsrührbehälter, 3 Produktdampfseparatoren, 3 Produktdestillationsbehälter und eine Wasserwäsche und Wasserkondensation. Für die Aufbereitung des Reaktionsöles und seiner konstanten Zusammensetzung ist zusätzlich ein Inertgasvakuumverdampfer (7) mit Zusatzelementen notwendig. Das Rohrbündel für die anschließende Umsetzung von der Mischung aus Alkanen zu Methanol, Methan und Wasserstoff im Röhrenbündeldruckreaktor (8) besteht aus einer sich von oben nach unten windenden Rohrleitung mit einem Durchmesser von 2 Zoll und einer Höhe von 3 Metern. Am unteren Ende ist eine Verbindungsleitung auf eine sich windende Rohrleitung von unten nach oben. Die beiden Teile des beheizten Röhrenbündeldruckreaktors (8) sind getrennt durch eine unten offene Trennwand, die den Gegenstrom des Thermoöls realisiert. Am Ende ist der Vorwärmer des Eingangsmischprodukts und ein Nachkühler mit Ventilator. Danach ist das Entspannungsventil mit anschließendem Zyklon für die Abscheidung des Katalysators angeordnet.

Die nach dem erfindungsgemäßen Verfahren hergestellte Mischung aus Alkanen wurde analysiert. Das Ergebnis der Analyse ist in Tabelle 1 wiedergegeben.

**Tab. 1 Analyse der Mischung aus Alkanen**

| **Parameter** | **Methode** | **Ergebnis** | **Einheit** |
|---|---|---|---|
| Dichte (15 °C) | DIN EN ISO 12185:1997 | 846,4 | kg/m3 |
| Kinetische Viskosität (40 °C) | DIN EN ISO 3104:2021 | 1,804 | mm2/s |
| Flash-Point | DIN EN ISO 2719:2021 | 41,0 | °C |
| CFPP | DIN EN 116:2018 | -16 | °C |
| Schwefelgehalt | DIN EN ISO 20884:2022 | 359 | mg/kg |
| Cetan-Nummer (ICN) | DIN EN 17155:2018 | 62,3 | - |
| Schwefelasche (775 °C) | ISO 3987:2010 | <0,005 | % (m/m) |
| Wassergehalt | DIN 51777 Verf. A: 2020 | 3564 | mg/kg |
| Gesamtverunreinigung | DIN EN 12662:1998 | <1 | mg/kg |
| Kupferstreifenkorrosion | DIN EN ISO 2160:1999 | 1 | Korr.° |
| Oxidationsstabilität | DIN EN 14112:2021 | 0,04 | h |
| Säurezahl | DIN EN 14104:2003 | 106 | mg KOH/g |
| Alkaligehalt (Na+K) | DIN EN 14538:2006 | <1,0 | mg/kg |
| Metallgehalt (Ca+Mg) | | <1,0 | mg/kg |
| Phosphorgehalt | DIN EN 14107:2003 | <4,0(<0,5) | mg/kg |
| Cloud-Point | DIN EN 23015:1994 | -10 | °C |

## Patentansprüche

1. Verfahren zur Herstellung einer Mischung aus Alkanen umfassend wenigstens die Schritte:
(A) Umsetzen von Biomasse mit wenigstens einem Gas, das Sauerstoff enthält, in Anwesenheit wenigstens eines Katalysators in der Flüssigphase bei einer Temperatur im Bereich von ≥ 270 bis ≤ 360 °C, bevorzugt bei 300 °C, um eine Mischung aus Reaktionsgas, Mischung aus Alkanen und Wasserdampf zu erhalten, und
(B) Abtrennen der Mischung aus Alkanen von Reaktionsgas und Wasserdampf durch Kondensation in einem Produktdampfseparator (2), um die Mischung aus Alkanen zu erhalten,
**dadurch gekennzeichnet, dass** Schritt (A) in einem Injektionsrührbehälter (1) erfolgt.

2. Verfahren nach Anspruch 1, **dadurch gekennzeichnet, dass** der Injektionsrührbehälter (1) einen Tank (1d), wenigstens einen Rührer (1a) und wenigstens zwei Einlässe (1b, 1c) für Gase umfasst, wobei der wenigstens eine Rührer (1a) im unteren Bereich des Tanks (1d) angeordnet ist.

3. Verfahren nach Anspruch 2, **dadurch gekennzeichnet, dass** einer der wenigstens zwei Einlässe (1b) oberhalb des Rührers (1a) angeordnet ist und einer der wenigstens zwei Einlässe (1c) unterhalb des Rührers (1a) angeordnet ist.

4. Verfahren nach Anspruch 3, **dadurch gekennzeichnet, dass** das wenigstens eine Gas, das Sauerstoff enthält, über den wenigstens einen ersten Einlass (1b) angesaugt wird, wobei das Gas vorgewärmt ist, und das wenigstens eine Gas, das Sauerstoff enthält, mit Druck über den wenigstens einen zweiten Einlass (1c) zugeführt wird.

5. Verfahren nach einem der Ansprüche 1 bis 4, **dadurch gekennzeichnet, dass** der wenigstens eine Katalysator ein Aluminium-Silikat, bevorzugt eine Kation-Aluminium-Silikat, ist.

6. Verfahren nach einem der Ansprüche 1 bis 5, **dadurch gekennzeichnet, dass** der Injektionsrührbehälter (1) wenigstens einen Auslass (1e) umfasst.

7. Verfahren nach einem der Ansprüche 1 bis 6, **dadurch gekennzeichnet, dass** die Mischung aus Reaktionsgas, Mischung aus Alkanen und Wasserdampf über den wenigstens einen Auslass (1e), einen Bogen (2a) und wenigstens einen Wärmeaustauscher (2b) dem Produktdampfseparator (2) zugeführt wird.

8. Verfahren nach einem der Ansprüche 1 bis 7, **dadurch gekennzeichnet, dass** die Kondensation in einem Produktdampfseparator (2) bei einer Temperatur im Bereich von ≥ 90 bis ≤ 110 °C, bevorzugt bei 100 °C, erfolgt.

9. Verfahren nach einem der Ansprüche 1 bis 8, **dadurch gekennzeichnet, dass** die Mischung aus Alkanen eine Mischung aus Alkanen mit der Formel CₙH₂ₙ₊₂ ist, wobei n = 14, 15, 16, 17, 18, 19, 20, 21, 22 oder 23 ist, bevorzugt ist n = 17.

10. Verfahren nach einem der Ansprüche 1 bis 9, **dadurch gekennzeichnet, dass** die Flüssigphase in einem Inertgasvakuumverdampfer (7) rezyklisiert wird, wobei ein fester Rückstand abgetrennt wird.

11. Mischung aus Alkanen mit der Formel CₙH₂ₙ₊₂, wobei n = 14, 15, 16, 17, 18, 19, 20, 21, 22 oder 23 ist, die nach dem Verfahren nach einem der Ansprüche 1 bis 10 erhalten wird.

12. Mischung aus Alkanen mit der Formel CₙH₂ₙ₊₂, wobei n = 14, 15, 16, 17, 18, 19, 20, 21, 22 oder 23 ist, die eine Dichte bei 15 °C im Bereich von ≥ 740 bis ≤ 850 kg/m³, bestimmt nach DIN EN ISO 12185:1997, und
einen Flammpunkt im Bereich von ≥ 30 bis ≤ 45 °C, bestimmt nach DIN EN ISO 2719:2021, und
einen Schwefelgehalt im Bereich von ≥ 5 bis ≤ 500 mg/kg, bestimmt nach DIN EN ISO 20884:2022, und
eine Säurezahl im Bereich von ≥ 100 bis ≤ 110 mg KOH/g, bestimmt nach DIN EN ISO 14104:2003, aufweist.

13. Verfahren zur Herstellung von Methanol oder Methan oder Wasserstoff, **dadurch gekennzeichnet, dass** die Mischung aus Alkanen gemäß Anspruch 11 oder 12 mit Wasser gemäß der Formel CₙH₂ₙ₊₂ + n × H₂O = n × CH₃OH umgesetzt wird, wobei n = 14, 15, 16, 17, 18, 19, 20, 21, 22 oder 23 ist, oder
die Mischung aus Alkanen gemäß Anspruch 11 oder 12 mit Wasser gemäß der Formel 2 CₙH₂ₙ₊₂ + 2 × 1,5 n × H₂O = 2n × CH₄ + n CO₂ umgesetzt wird, wobei n = 14, 15, 16, 17, 18, 19, 20, 21, 22 oder 23 ist, oder
die Mischung aus Alkanen gemäß Anspruch 11 oder 12 mit Wasser gemäß der Formel CₙH₂ₙ₊₂ + 2n × H₂O = 3n × H₂ + n CO₂ umgesetzt wird, wobei n = 14, 15, 16, 17, 18, 19, 20, 21, 22 oder 23 ist.

14. Vorrichtung zur Herstellung einer Mischung aus Alkanen umfassend
wenigstens einen Injektionsrührbehälter (1), der einen Tank (1d), wenigstens einen Rührer (1a), wenigstens einen Auslass (1e) und wenigstens zwei Einlässe (1b, 1c) für Gase umfasst, wobei der wenigstens eine Rührer (1a) im unteren Bereich des Tanks angeordnet ist, wenigstens einer der zwei Einlässe (1b) oberhalb des Rührers (1a) angeordnet ist, und einer der wenigstens zwei Einlässe (1c) unterhalb des Rührers (1a) angeordnet ist, und
einen Produktdampfseparator (2),
wobei die Wärmeaustauscher (2b) zwischen dem wenigstens einen Auslass (1e) und dem Produktdampfseparator (2) angeordnet sind.

15. Vorrichtung nach Anspruch 14, **dadurch gekennzeichnet, dass** das wenigstens eine Gas, das Sauerstoff enthält, über den wenigstens einen ersten Einlass (1b) angesaugt wird, wobei das Gas durch Wärmeaustauscher (2b) vorgewärmt ist, und das wenigstens eine Gas, das Sauerstoff enthält, mit Druck über den wenigstens einen zweiten Einlass (1c) zugeführt wird, wobei der zweite Einlass (1c) mit einem Wärmeaustauscher und Luftverdichter (1f) verbunden ist.
